# FASCICULE DE BREVET EUROPEEN

(11) **EP 0 770 373 B1**
(45) Date de publication et mention de la délivrance du brevet: **28.01.1998**
(21) Numéro de dépôt: 96401952.5
(22) Date de dépôt: 12.09.1996
(51) Int. Cl.: A61K 7/00, A61K 7/15, A61K 7/48, A61K 7/06

(54) **Composition cosmétique contenant un monoester d'acide en C4-C10 et d'alcool en C16-C18 et des particules creuses**
Kosmetische Zusammensetzung, die einen Monoester einer C4-C10 Carbonsäure mit einer C16-C18 Alkohol und hohlen Teilchen enthält
Cosmetic composition containing a monoester of a C4-C10 acid with a C16-C18 alcohol and hollow particles

(30) Priorité: 25.10.1995 FR 9512584
(43) Date de publication de la demande: 02.05.1997
(73) Titulaire: L'OREAL, 75008 Paris (FR)
(72) Inventeur: Lavaud, Brigitte, 75016 Paris (FR)
(74) Mandataire: Andral, Christophe André Louis

(56) Documents cités:
- EP-A- 0 447 286
- EP-A- 0 452 202
- EP-A- 0 463 962
- DE-A- 4 221 914
- DATABASE WPI Week 4485 Derwent Publications Ltd., London, GB; AN 85-272732 XP002007166 "Globular polymer-contg., cosmetic - can be applied easily , shows good adhesiveness , and gives soft touch and transparency" & JP 60 184 004 A (POLA KASEI KOGYO) , 19 Septembre 1985

## Description

La présente invention concerne une nouvelle composition cosmétique pour la peau, y compris le cuir chevelu, plus spécialement pour le rasage, mais qui peut aussi servir comme nettoyant visage (nettoyant scrub) ou déodorant, et comprenant, **(a)** au moins un ester spécifique qui est un monoester d'acide aliphatique en C₄-C₁₀ et d'alcool aliphatique en C₁₆-C₁₈, et **(b)** des particules creuses présentant une granulométrie moyenne allant de 1 µm à 300 µm.

Les produits utilisés par l'homme pour éliminer les poils de son corps à des fins esthétiques ou hygiéniques sont connus et variés, depuis le savon à raser en poudre, en barre ou en pain, jusqu'aux crèmes et mousses à raser et aux gels aqueux de rasage (se reporter à cet égard à l'article intitulé 〈〈 Shaving Preparations - Origin of Shaving and Sales Trends 〉〉, par Robert E. Sauté, publié dans l'ouvrage 〈〈 The Chemistry and Manufacture of Cosmetics 〉〉_{,} Second Edition-1975-Vol IV-chapitre 64-pages 1313 à 1341, de Maison G. de Navarre).

A côté des gels de rasage traditionnels, on connaît également les gels de rasage à moussage différé (ou à effet de mousse différé) qui ont été plus précisément décrits dans de nombreux brevets tels que, par exemple, US 3 541 581, US 4 405 489, et FR 2 595 943.

Ces gels à effet de mousse différé sont des compositions généralement conditionnées sous pression dans un dispositif aérosol qui délivre, sous l'effet d'un propulseur, des gels non moussants dans des conditions statiques, mais qui, sous l'action mécanique due à l'étalement du produit sur la peau, engendrent spontanément et presque instantanément une mousse sur cette dernière.

Les gels de rasage à moussage différé doivent réunir de nombreuses caractéristiques qui rendent leur mise au point particulièrement difficile.

Tout d'abord, le gel délivré dans la main doit présenter une rhéologie appropriée (rigidité, consistance). En outre, à l'étalement sur la peau du visage, ce gel doit pouvoir développer très rapidement une mousse, elle-même susceptible de prendre du volume sans toutefois produire des petits amas de mousse rigide. Enfin, la mousse engendrée par le gel doit être dotée de bonnes qualités cosmétiques, telles que l'hydratation, l'apport de douceur, et l'absence d'effet collant et/ou d'effet filant sur la peau.

Malheureusement, les compositions de gel de rasage à moussage différé de l'art antérieur ne remplissent généralement pas l'ensemble de ces conditions.

Pour faciliter la mise en oeuvre et augmenter la stabilité de tels gels, on a déjà proposé d'y introduire des alcanolamides d'acides gras en C₁₂-C₁₈, ou des alcools gras éthoxylés, ou bien encore du dipelargonate de propylène glycol. Cependant, là encore, les gels obtenus n'ont pas réuni toutes les propriétés ci-dessus énoncées et ne se sont donc pas encore révélés totalement satisfaisants.

Les inconvénients mentionnés ci-dessus sont aussi vrais pour d'autres types de gels ainsi que pour des mousses à raser, des produits nettoyant visage et des déodorants.

La présente invention vise à proposer des compositions cosmétiques et plus spécialement de rasage, et plus particulièrement encore des gels de rasage à moussage différé, des crèmes et des mousses à raser, présentant des propriétés améliorées.

Ainsi, après de nombreuses recherches menées sur la question, la Demanderesse a maintenant découvert, de façon totalement inattendue et surprenante, qu'en ajoutant un monoester d'acide aliphatique en C₄-C₁₀ et d'alcool aliphatique en C₁₆-C₁₈, et des particules creuses présentant une granulométrie moyenne allant de 1 µm à 300 µm, à une composition cosmétique pour la peau, on pouvait obtenir une composition et notamment de rasage, présentant des performances et/ou des qualités améliorées, et en particulier toutes les caractéristiques recherchées décrites précédemment.

Cette découverte est à la base de la présente invention.

La présente invention a ainsi pour objet une nouvelle composition cosmétique pour la peau, y compris le cuir chevelu, caractérisée par le fait qu'elle comprend, **(a)** au moins un monoester d' acide aliphatique en C₄-C₁₀ et d'alcool aliphatique en C₁₆-C₁₈, et **(b)** des particules creuses présentant une granulométrie moyenne allant de 1 µm à 300 µm.

Elle a aussi pour objet une composition de rasage du type comprenant un savon hydrosoluble, et qui est caractérisée par le fait qu'elle comprend en outre, **(a)** au moins un monoester d' acide aliphatique en C₄-C₁₀ et d'alcool aliphatique en C₁₆-C₁₈, et **(b)** des particules creuses présentant une granulométrie moyenne allant de 1 µm à 150 µm.

Elle vise également l'utilisation d'une composition telle que décrite ci-avant pour le rasage des poils et/ou des cheveux présents sur la peau humaine ou animale. Elle vise aussi l'utilisation d'une telle composition,
comme, ou pour la fabrication de, une composition cosmétique déodorante.

Le ou les monoesters d'acides aliphatiques en C₄-C₁₀ et d'alcools aliphatiques en C₁₆-C₁₈ qui peuvent être utilisés selon l'invention sont de préférence choisis parmi les monoesters d'acides aliphatiques saturés linéaires ou ramifiés en C₄-C₁₀ et d'alcools aliphatiques saturés linéaires en C₁₆-C₁₈.

Des monoesters de ce type plus particulièrement préférés selon la présente invention sont l'ester de l'acide heptanoique et de l'alcool stéarylique (ou heptanoate de stéaryle), l'ester de l'acide octanoique et de l'alcool stéarylique (ou octanoate de stéaryle), ou encore des mélanges heptanoate / octanoate de stéaryle, en particulier le mélange heptanoate (67%) / octanoate (33%) de stéaryle vendu par exemple par la société CRODA sous la dénomination commerciale CRODAMOL W, ou par la société STEARINERIES DUBOIS sous la dénomination commerciale DUB SOLIDE.

Les particules creuses qui peuvent être utilisées selon l'invention présentent de façon avantageuse une granulométrie moyenne allant de 5 µm à 200 µm et de préférence de 10 µm à 100 µm et mieux de 15 µm à 60 µm. Dans le cas particulier d'une composition de rasage, la granulométrie est choisie de 1 à 150 µm et de préférence de 10 à 100 µm et mieux de 15 à 60 µm.

Les particules sont avantageusement réalisées en matériaux thermoplastiques comme les polyamides tels que le Nylon, les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

Comme particules de Nylon, on peut utiliser les particules d' 〈〈 Orgasol 〉〉 vendues par la société Atochem. Ces particules sont des sphères pleines, poreuses, de diamètre allant de 5 µm à 60 µm.

De préférence, les particules sont des particules creuses d'un copolymère expansé, de chlorure de vinylidène et d'acrylonitrile, ou d'un terpolymère expansé de chlorure de vinylidène, d'acrylonitrile et de méthacrylate. On peut par exemple utiliser un terpolymère contenant : de 0 % à 60% de motifs dérivés du chlorure de vinylidène, de 20% à 90% de motifs dérivés d'acryonitrile et de 0% à 50% motifs dérivés d'un monomère acrylique ou styrénique, la somme des pourcentages (en poids) étant égale à 100. Ces particules peuvent être sèches ou hydratées et sont, par exemple, celles vendues sous la marque EXPANCEL par la société Nobel Casco, et en particulier sous les références 551 DE (granulométrie d'environ 50 µm et masse volumique d'environ 35 kg/m³), 551 DE 12 (granulométrie d'environ 12 µm et masse volumique d'environ 40 kg/m³), 551 DE 20 (granulométrie d'environ 30 µm et masse volumique d'environ 65 kg/m³), 551 DE 50 (granulométrie d'environ 40 µm), 461 DE 50 et 642 WE 50 de 50 µm de granulométrie environ, 551 DE 80 (granulométrie de 80 µm environ).

On peut aussi utiliser des particules de ce même terpolymère ayant une granulométrie d'environ 18 µm et une masse volumique d'environ 60 à 90 kg/m³, ou encore de granulométrie d'environ 34 µm et de masse volumique d'environ 20 kg/m³.

On peut encore utiliser des particules de copolymère non expansé de chlorure de vinylidène et d'acrylonitrile, ou de terpolymère non expansé de chlorure de vinylidène, d'acrylonitrile et de méthacrylate, comme celles vendues sous la marque EXPANCEL avec la référence 551 DU 10 (granulométrie d'environ 10 µm), ou 461 DU 15 (granulométrie d'environ 15 µm).

Comme autre particules creuses polymériques utilisables selon l'invention, on peut encore citer les polymères et les copolymères obtenus à partir des esters (acétate ou lactate de vinyle), ou acides (itaconique, citraconique, maléique, fumarique) [voir à cet effet le document JP-A-2-112304].

Selon une autre caractéristique avantageuse de l'invention, les particules se présentent sous forme de billes. Il est toutefois possible d'utiliser des particules ayant la forme de fibres ou d'aiguilles.

Les particules vendues sous la marque Expancel, selon l'invention, peuvent être obtenues, par exemple, selon les procédés des brevets et demandes de brevet EP-56 219, EP-348 372, EP-486 080, EP-320 473, EP-112 807, US-3 615 972. La cavité interne des particules contient en principe un gaz qui peut être de l'air, de l'azote ou un hydrocarbure comme l'isobutane ou l'isopentane.

Les autres constituants rentrant dans la composition de l'invention et en particulier des produits de rasage, sont des produits déjà connus en soi dans l'application considérée (voir à cet égard la référence de Robert E. Sauté citée ci-avant), et plus précisément pour les gels de rasage à effet de mousse différé, (voir à cet égard EP-A-259 843, US 3 541 581 et FR 2 595 943). Pour les compositions de nettoyage et de scrub, on peut se référer au document
FR 2 700 952, et pour les produits déodorants, on peut se référer à l'ouvrage 〈〈 Cosmetic Science and Technology Series 〉〉 - 1988 / Volume 7.

Le savon hydrosoluble est de préférence un sel hydrosoluble d'acide gras. De tels savons sont bien connus de l'art antérieur, existent dans le commerce ou peuvent être préparés selon les méthodes conventionnelles, par exemple par réaction d'une base, telle que la triéthanolamine, la soude ou la potasse, directement sur un acide gras, tel qu'un acide gras saturé ou non saturé en C₁₀ à C₂₂, ou des mélanges de ces acides. Des savons préférés selon l'invention comprennent les stéarates, myristates et palmitates hydrosolubles, tels que les savons solubles des acides stéarique ou palmitique commerciaux. Les sels de sodium et de potassium de ces acides et leurs mélanges sont utilisés de façon avantageuse pour les savons, les crèmes et les mousses à raser, et les sels de triéthanolamine de ces acides sont plus particulièrement préférés pour les gels de rasage. Par ailleurs, il est bien connu que le produit commercial vendu sous le nom d'acide stéarique peut être un mélange d'acides stéarique et palmitique. Le terme 〈〈stéarates〉〉 désigne les savons d'acide stéarique du commerce, mais peut également désigner les savons d'acide stéarique pur.

Un savon hydrosoluble particulièrement préféré selon l'invention pour les gels de rasage à effet de mousse différé est le palmitate de triéthanolamine.

Un polymère gélifiant hydrosoluble peut être utilisé selon l'invention pour stabiliser la composition ou pour ajuster sa consistance à la valeur désirée. Il peut alors être choisi parmi les hydroxyalkylcelluloses hydrosolubles ou les gommes naturelles telles que la gomme de xanthane. Les hydroxyalkylcelluloses sont fabriquées à partir d'une alkyl cellulose et d'un oxyde d'alkylène tel que l'oxyde d'éthylène ou l'oxyde de propylène. Des produits de ce type sont vendus sous les marques 〈〈KLUCEL〉〉 et 〈〈NATROSOL〉〉 dans une gamme de viscosités variées.
Un polymère hydrosoluble plus particulièrement préféré selon la présente invention est une hydroxypropylcellulose vendue sous la marque 〈〈KLUCEL H〉〉 ou 〈〈KLUCEL MF〉〉 par la société AQUALON, une hydroxyéthylcellulose vendue sous la marque 〈〈NATROSOL 250 HHR〉〉 par la société AQUALON, ou un mélange de ces deux celluloses.

L'un des avantages attachés à la présente invention est que l'on peut réduire, voire supprimer, la quantité d'épaississant cellulosique dont les proportions trop élevées sont à l'origine de l'inconvénient du collant sur la peau.

Dans les gels de rasage à effet de mousse différé, l'agent de moussage différé est liquide ou liquéfiable, et volatil à la température de la peau. Il comprend les hydrocarbures aliphatiques saturés comportant de 4 à 6 atomes de carbone, tels que les butanes, n-butane ou isobutane, les pentanes, tels que le n-pentane ou l'isopentane, ou encore l'hexane. Des mélanges de ces hydrocarbures peuvent être utilisés pour obtenir la pression de vapeur désirée.

Un agent de moussage différé particulièrement bien adapté pour régler la pression du gel en vue d'obtenir les qualités de mousse requises pour la présente invention est le mélange isopentane (75% en poids) / isobutane (25% en poids).

L'eau présente dans la composition de rasage selon l'invention participe aux qualités recherchées de la mousse, sert à humidifier la peau et à assurer un rasage convenable.

Dans les compositions de rasage selon la présente invention, le monoester d'acide aliphatique en C₄-C₁₀ et d'alcool aliphatique en C₁₆-C₁₈ est généralement présent dans des concentrations pondérales (ramenées à l'ensemble de la composition) allant environ de 0,1 à 5%, de préférence allant environ de 1,5 à 3%, et les particules creuses sont généralement présentes dans des concentrations pondérales allant environ de 0,005 à 5%, de préférence allant environ de 0,01 à 2%, ce qui correspond à une concentration en volume allant environ de 0,07% à 150%, et de préférence allant environ de 0,15% à 60%.

Le savon hydrosoluble est généralement présent dans des concentrations pondérales allant environ de moins de 5% à plus de 95% suivant l'application considérée, jusqu'à environ 95% et plus dans les savons solides, jusqu'à environ 40% dans les crèmes et les mousses, et jusqu'à environ 20% dans les gels. De façon avantageuse, on utilise de préférence dans les gels à effet de mousse différé une quantité de savon allant d'environ 10 à 15%.

Le polymère gélifiant hydrosoluble peut être présent dans des concentrations pondérales allant d'environ 0 à 3%, de préférence d'environ 0 à 1% selon la viscosité du produit employé.

L'agent de moussage différé (dans le cas des gels de rasage à moussage différé) est généralement présent dans des concentrations pondérales allant d'environ 0,5 à 10%, de préférence d'environ 2 à 5%.

L'eau est généralement présente dans des concentrations pondérales allant de 0% pour les savons solides à 90% pour les gels, et de façon préférentielle pour les gels entre environ 60 et 90%.

Les compositions selon l'invention peuvent encore contenir d'autres ingrédients ou actifs bien connus dans le domaine des produits cosmétiques pour l'application choisie, et notamment pour les applications de rasage, on peut citer par exemple, des hydratants, et parmi eux la glycérine et le sorbitol, des agents apaisants tels que l'allantoine ou l'α-bisabolol, des lubrifiants tels que des silicones ou des polydécènes, des agents surgraissants tels que la lanoline ou ses dérivés, des émollients comme le polyéthylèneglycol, le polypropylèneglycol, le benzoate d'alcools en C₁₂/C₁₅ ou les stéarates de glycérol et de polyéthylèneglycol, des agents opacifiants tels que l'oxyde de titane, des agents tensio-actifs, des agents stabilisants, des vitamines, des solvants, des colorants, parfums et conservateurs.

Bien entendu, l'homme de l'art veillera à choisir le ou les éventuels composés complémentaires mentionnés ci-avant, de manière telle que les propriétés avantageuses attachées intrinsèquement à la composition cosmétique selon l'invention ne soient pas, ou substantiellement pas, altérées par la ou les adjonctions envisagées.

Les crèmes et les gels selon l'invention peuvent être conditionnés sous forme de tubes ou d'aérosols, et les mousses en aérosol, selon des techniques bien connues de l'homme de l'art et en particulier pour les gels à effet de mousse différé comme décrit dans le brevet US 3 541 581.

Les dispositifs aérosol peuvent être à simple ou à double enveloppe.

Dans les dispositifs aérosol à double enveloppe, le système propulseur est séparé du gel ou de la crème conforme à l'invention. En effet, on introduit par exemple le gel ou la crème conforme à l'invention dans la partie centrale de l'aérosol à double enveloppe, on introduit le propulseur dans l'enveloppe extérieure qui est séparée de la partie centrale par un diaphragme qui est une membrane plastique compressible. Dans de tels dispositifs à double enveloppe, le système propulseur peut alors être constitué d'un gaz condensable tel qu'un hydrocarbure comme le propane, le butane, l'isobutane, l'isopentane, les hydrocarbures halogénés, ou le diméthyléther, ou encore les mélanges de ces composés. Il peut également être constitué d'un mélange de ces agents propulseurs condensables avec des gaz non condensables tels que l'oxyde nitreux ou l'azote.

Dans les dispositifs à simple enveloppe, le système propulseur est uniquement constitué de gaz non condensables, insolubles dans le gel conforme à l'invention, tel que l'azote, l'argon, le néon, le krypton, le xénon, l'hélium, le radon, l'oxyde nitreux ou le gaz carbonique. De tels dispositifs sont équipés d'un tube plongeur à la partie supérieure de laquelle on introduit ledit système propulseur. Dans ce cas, le système propulseur non condensable et insoluble dans la composition conforme à l'invention agit comme un piston et expulse la composition par le tube plongeur.

Dans les aérosols, la composition à l'état pressurisé (ou 〈〈 jus 〉〉) représente avantageusement de 90 à 98% du poids de l'ensemble de la composition, et le système propulseur de 2 à 10% de ce poids.

Des exemples concrets, mais nullement limitatifs, illustrant l'invention vont maintenant être donnés.

### EXEMPLE 1:

On a préparé un gel de rasage à moussage différé de la façon suivante : Dans la partie centrale d'un dispositif aérosol à double enveloppe, on a introduit (i) 96,5 grammes de la composition suivante :

| | |
|---|---|
| Acide palmitique | 12,5 g |
| Triéthanolamine | 9,2 g |
| Glycérine | 3,9 g |
| Hydroxypropylcellulose (Klucel MF de la société AQUALON) | 0,1 g |
| Polyéthylèneglycol (Polyox WSR 205 de la société AMERCHOL) | 0,2 g |
| Heptanoate (67%) / octanoate (33%) de stéaryle (Dub Solide de la société STEARINERIES DUBOIS) | 1,9 g |
| Microsphères expansées de copolymère chlorure de vinylidène/acrylonitrile contenant de l'isobutane vendu sous la marque Expancel 551 DE 20 par la société Nobel Casco | 0,05 g |
| Parfum, colorant | qs |
| Eau déminéralisée qsp | 100, g |

et (ii) 3,5 grammes d'un agent de moussage différé constitué d'un mélange isopentane (75%poids) / isobutane (25% poids).

On a fermé la partie centrale. On a procédé ensuite à la pressurisation du mélange obtenu ci-dessus ou 〈〈 jus 〉〉, en introduisant à la partie inférieure de la double enveloppe, et par l'opercule du fond, 4 à 10 grammes d'un propulseur constitué d'un mélange (pondéral) isobutane (55%), butane (22%), propane (23%) vendu sous la dénomination Aérogaz 3,2N par la société ELF AQUITAINE.

L'aérosol sert est alors prêt à être utilisé. Lorsqu'il est actionné, il délivre dans la main un gel d'une rhéologie telle qu'elle lui confère une rigidité lui permettant de tenir dans la main et de s'étaler avec facilité de façon homogène sur la peau du visage sans engendrer de petits amas de mousse. Sous l'action mécanique de l'étalement sur la peau, le gel développe rapidement une mousse, douce à l'application, onctueuse et régulière, qui ne colle pas à la peau et ne file pas. Après rasage, la peau est douce et lisse.

### EXEMPLE 2:

On a préparé une crème à raser de composition suivante :

| | |
|---|---|
| Acide stéarique (C16/C18 50/50 ) | 2,75 g |
| Triéthanolamine | 0,45 g |
| Glycérine | 5 g |
| Stéarate de glycérol et stéarate de polyéthylèneglycol 100 vendu par la société I.C.I. sous la dénomination Arlacel 165 | 5 g |
| Alcool stéarylique | 5 g |
| Heptanoate (67%) / octanoate (33%) de stéaryle (Dub Solide de la société STEARINERIES DUBOIS) | 5 g |
| Microsphères expansées de copolymère chlorure de vinylidène/acrylonitrile/méthacrylate de méthyle contenant de l'isobutane vendu sous la marque Expancel 551 DE par la société Nobel Casco | 0,1 g |
| Oxyde de titane traité par du stéarate d'aluminum/alumine | 1 g |
| Benzoate d'alcools en C₁₂/C₁₅ | 8 g |
| agent stabilisant : hexadécylphosphate de sodium | 1 g |
| α-bisabolol | 0,1 g |
| Parfum, conservateurs | qs |
| Eau déminéralisée qsp | 100 g |

Les propriétés cosmétiques de la crème sont comparables à celles du gel de l'exemple 1.

### EXEMPLE 3 :

On a préparé une mousse à raser de la façon suivante :
Dans un bidon aérosol, on a introduit par le col, (i) 96 grammes de la composition suivante :

| | |
|---|---|
| Acide stéarique (C16/C18 50/50) | 7,9 g |
| Triéthanolamine | 2,9 g |
| Glycérine | 2,5 g |
| Acide gras de coprah | 0,6 g |
| KOH en solution aqueuse à 50% | 0,4 g MA* |
| Heptanoate (67%) / octanoate (33%) de stéaryle (Dub Solide de la société STEARINERIES DUBOIS) | 0,5 g |
| Microsphères expansées de copolymère chlorure de vinylidène/acrylonitrile/méthacrylate de méthyle contenant de l'isobutane vendu sous la marque Expancel 551 DE 20 par la société Nobel Casco | 0,3 g |
| Monolaurate de sorbitan à 20 moles d'oxyde d'éthylène | 0,96 g |
| Polymère cationique (copolymère chlorure de diméthyldiallylammonium/acrylamide 50/50 en solution aqueuse à 8% MA, vendu sous la dénomination commerciale Merquat S par la société Merck | 0,23 g MA* |
| Mélange de cocoate et de lanolate d'isopropanolamine en solution aqueuse à 47% | 0,9 g MA* |
| Parfum, conservateurs | qs |
| Eau déminéralisée qsp | 100 g |

| | |
|---|---|
| * MA désigne Matière Active. | |

Après pose de la valve et sertissage sur le bidon, on a introduit par la valve, (ii) 4 grammes d'un propulseur constitué d'un mélange (pondéral) isobutane (55%), butane (22%), propane (23%) vendu sous la dénomination Aérogaz 3,2N par la société ELF AQUITAINE.

Les propriétés cosmétiques de la mousse à raser sont comparables à celles du gel de l'exemple 1.

### EXEMPLE 4 :

On a préparé un stick déodorant de composition suivante :

| | |
|---|---|
| Alcool butylique à 14 moles d'oxyde de propylène | 15 g |
| Palmitate d'isopropyle | 5 g |
| Heptanoate (67%) / octanoate (33%) de stéaryle (Dub Solide de la société STEARINERIES DUBOIS) | 4 g |
| Alcool stéarylique | 19 g |
| Huile de ricin hydrogénée | 2 g |
| Hydroxychlorure d'aluminium anhydre micronisé | 15 g |
| Talc (silicate de magnésium) | 2 g |
| Microsphères expansées de copolymère chlorure de vinylidène/acrylonitrile/méthacrylate de méthyle contenant de I'isobutane vendu sous la marque Expancel 551 DE 20 par la société Nobel Casco | 0,7 g |
| Conservateur, parfum, | qs |
| Huile de silicone : | |
| D.C.345 Fluid de la société Dow Corning) qsp | 100 g |

Ce stick laisse la peau douce et non collante.

## Revendications

1. Composition cosmétique pour la peau et le cuir chevelu caractérisée par le fait qu'elle comprend **(a)** au moins un monoester d'acide aliphatique en C₄-C₁₀ et d'alcool aliphatique en C₁₆-C₁₈, et **(b)** des particules creuses présentant une granulométrie moyenne allant de 1 µm à 300 µm.

2. Composition de rasage du type comprenant un savon hydrosoluble, caractérisée par le fait qu'elle comprend en outre, **(a)** au moins un monoester d'acide aliphatique en C₄-C₁₀ et d'alcool aliphatique en C₁₆-C₁₈, et **(b)** des particules creuses présentant une granulométrie moyenne allant de 1 µm à 150 µm.

3. Composition selon les revendications 1 ou 2, caractérisée par le fait que ledit monoester, ou composé **(a)**, est choisi parmi les heptanoates de stéaryle, les octanoates de stéaryle ou leurs mélanges.

4. Composition selon l'une quelconque des revendications 1 à 3, caractérisée par le fait que ledit monoester est un mélange heptanoate / octanoate de stéaryle.

5. Composition selon l'une quelconque des revendications précédentes, caractérisée par le fait que lesdites particules creuses, ou composé **(b)**, sont formées d'un matériau thermoplastique.

6. Composition selon l'une quelconque des revendications précédentes, caractérisée par le fait que les particules creuses sont réalisées en un matériau choisi parmi le nylon, les polymères et copolymères de polychlorure de vinylidène, d'acrylonitrile et/ou d'un monomère acrylique ou styrénique.

7. Composition selon l'une quelconque des revendications précédentes, caractérisée par le fait que les particules creuses sont réalisées en un matériau choisi parmi un copolymère de chlorure de vinylidène, d'acrylonitrile et/ou d'un monomère acrylique ou styrénique, expansé.

8. Composition selon l'une quelconque des revendications précédentes, caractérisée par le fait que lesdites particules creuses présentent une granulométrie moyenne allant de 10 µm à 100 µm.

9. Composition selon l'une quelconque des revendications précédentes, caractérisée par le fait que ledit savon est choisi parmi les sels de l'acide stéarique ou myristique ou palmitique, ou encore d'un mélange de ces acides.

10. Composition selon l'une quelconque des revendications précédentes, caractérisée par le fait que ledit ester est présent dans des concentrations pondérales allant de 0,1 à 5% et de préférence de 1,5 à 3% en poids par rapport au poids total de la composition.

11. Composition selon l'une quelconque des revendications précédentes, caractérisée par le fait que lesdites particules creuses représentent de 0,005% à 5% et de préférence de 0,01% à 2% du poids total de la composition.

12. Composition selon l'une quelconque des revendications précédentes, caractérisée par le fait qu'elle se présente sous la forme d'un savon solide ou liquide, d'une crème, d'un gel, ou d'une mousse.

13. Composition selon l'une quelconque des revendications précédentes, caractérisée par le fait qu'il s'agit d'un gel aqueux de rasage à effet de mousse différé comprenant en outre un agent de moussage volatil 〈〈 à effet différé 〉〉, choisi dans le groupe constitué des butanes, des pentanes, des hexanes et de leurs mélanges.

14. Gel selon la revendication 13, caractérisé par le fait que ledit agent de moussage différé volatil est un mélange d'isopentane et d'isobutane.

15. Gel selon les revendications 13 ou 14 caractérisé par le fait que ledit agent de moussage différé est présent dans des concentrations allant de 0,5 à 10% et de préférence de 2 à 5% en poids par rapport au poids total de la composition.

16. Gel selon les revendications 12 à 15, caractérisé par le fait qu'il se présente dans un état pressurisé sous la forme d'un aérosol.

17. Utilisation d'une composition telle que définie à l'une quelconque des revendications précédentes pour le rasage des poils ou des cheveux.

18. Utilisation d'une composition telle que définie à l'une quelconque des revendications 1 à 12, comme, ou pour la fabrication de, une composition cosmétique déodorante.

## Claims

1. Cosmetic composition for the skin and the scalp, characterized in that it comprises **(a)** at least one monoester of a C₄-C₁₀ aliphatic acid and of a C₁₆-C₁₈ aliphatic alcohol and **(b)** hollow particles exhibiting a mean particle size ranging from 1 µm to 300 µm.

2. Shaving composition of the type comprising a water-soluble soap, characterized in that it additionally comprises **(a)** at least one monoester of a C₄-C₁₀ aliphatic acid and of a C₁₆-C₁₈ aliphatic alcohol and **(b)** hollow particles exhibiting a mean particle size ranging from 1 µm to 150 µm.

3. Composition according to Claims 1 and 2, characterized in that the said monoester, or compound **(a)**, is chosen from stearyl heptanoates, stearyl octanoates or their mixtures.

4. Composition according to any one of Claims 1 to 3, characterized in that the said monoester is a stearyl heptanoate/octanoate mixture.

5. Composition according to any one of the preceding claims, characterized in that the said hollow particles, or compound **(b)**, are formed from a thermoplastic material.

6. Composition according to any one of the preceding claims, characterized in that the hollow particles are made of a material chosen from nylon or polymers and copolymers of polyvinylidene chloride, of acrylonitrile and/or of an acrylic or styrene monomer.

7. Composition according to any one of the preceding claims, characterized in that the hollow particles are made of a material chosen from an expanded copolymer of vinylidene chloride, of acrylonitrile and/or of an acrylic or styrene monomer.

8. Composition according to any one of the preceding claims, characterized in that the said hollow particles exhibit a mean particle size ranging from 10 µm to 100 µm.

9. Composition according to any one of the preceding claims, characterized in that the said soap is chosen from salts of stearic or myristic or palmitic acid or else of a mixture of these acids.

10. Composition according to any one of the preceding claims, characterized in that the said ester is present in weight concentrations ranging from 0.1 to 5% and preferably from 1.5 to 3% by weight with respect to the total weight of the composition.

11. Composition according to any one of the preceding claims, characterized in that the said hollow particles represent from 0.005% to 5% and preferably from 0.01% to 2% of the total weight of the composition.

12. Composition according to any one of the preceding claims, characterized in that it is provided in the form of a solid or liquid soap, of a cream, of a gel or of a foam.

13. Composition according to any one of the preceding claims, characterized in that it relates to an aqueous shaving gel with a delayed foam effect additionally comprising a volatile foaming agent "with a delayed effect" chosen from the group consisting of butanes, pentanes, hexanes and their mixtures.

14. Gel according to Claim 13, characterized in that the said volatile delayed-foaming agent is a mixture of isopentane and isobutane.

15. Gel according to Claims 13 and 14, characterized in that the said delayed-foaming agent is present in concentrations ranging from 0.5 to 10% and preferably from 2 to 5% by weight with respect to the total weight of the composition.

16. Gel according to Claims 12 to 15, characterized in that it is provided in a pressurized state in the form of an aerosol.

17. Use of a composition as defined in any one of the preceding claims for shaving hair.

18. Use of a composition as defined in any one of Claims 1 to 12 as, or for the manufacture of, a deodorant cosmetic composition.

## Patentansprüche

1. Kosmetische Zusammensetzung für die Haut und die Kopfhaut, dadurch gekennzeichnet, daß sie (a) mindestens einen Monoester einer aliphatischen C₄₋₁₀-Säure und eines aliphatischen C₁₆₋₁₈-Alkohols und (b) Hohlpartikel, die eine mittlere Teilchengröße von 1 µm bis 300 µm aufweisen, enthält.

2. Zusammensetzung zum Rasieren, die eine wasserlösliche Seife enthält, dadurch gekennzeichnet, daß sie ferner (a) mindestens einen Monoester einer aliphatischen C₄₋₁₀-Säure und eines aliphatischen C₁₆₋₁₈-Alkohols und (b) Hohlpartikel, die eine mittlere Teilchengröße von 1 µm bis 150 µm aufweisen, enthält.

3. Zusammensetzung nach Anspruch 1 oder 2, dadurch gekennzeichnet, daß der Monoester oder die Verbindung (a) unter Stearylheptanoat, Stearyloctanoat oder Gemischen dieser Verbindungen ausgewählt ist.

4. Zusammensetzung nach einem der Ansprüche 1 bis 3, dadurch gekennzeichnet, daß der Monoester ein Gemisch von Stearylheptanoat und Stearyloctanoat ist.

5. Zusammensetzung nach einem der vorhergehenden Ansprüche, dadurch gekennzeichnet, daß die Hohlpartikel oder die Verbindung (b) aus einem thermoplastischen Material bestehen.

6. Zusammensetzung nach einem der vorhergehenden Ansprüche, dadurch gekennzeichnet, daß die Hohlpartikel in einem Material ausgeführt sind, das unter Nylon, den Polymeren und Copolymeren von Vinylidenchlorid, Acrylnitril und/oder einem Acrylmonomer oder einem Styrolmonomer ausgewählt ist.

7. Zusammensetzung nach einem der vorhergehenden Ansprüche, dadurch gekennzeichnet, daß die Hohlpartikel in einem Material ausgeführt sind, das unter einem expandierten Copolymer von Vinylidenchlorid, Acrylnitril und/oder einem Acrylmonomer oder einem Styrolpolymer ausgewählt ist.

8. Zusammensetzung nach einem der vorhergehenden Ansprüche, dadurch gekennzeichnet, daß die Hohlpartikel eine mittlere Teilchengröße von 10 µm bis 100 µm aufweisen.

9. Zusammensetzung nach einem der vorhergehenden Ansprüche, dadurch gekennzeichnet, daß die Seife unter den Salzen der Stearinsäure, der Myristinsäure oder der Palmitinsäure oder auch den Salzen eines Gemischs dieser Säuren ausgewählt ist.

10. Zusammensetzung nach einem der vorhergehenden Ansprüche, dadurch gekennzeichnet, daß der Ester in Konzentrationen von 0,1 bis 5 Gew.-% und vorzugsweise von 1,5 bis 3 Gew.-% vorliegt, bezogen auf das Gesamtgewicht der Zusammensetzung.

11. Zusammensetzung nach einem der vorhergehenden Ansprüche, dadurch gekennzeichnet, daß die Hohlpartikel 0,005 % bis 5 % und vorzugsweise 0,01 % bis 2 % des Gesamtgewichts der Zusammensetzung ausmachen.

12. Zusammensetzung nach einem der vorhergehenden Ansprüche, dadurch gekennzeichnet, daß sie in Form einer festen oder flüssigen Seife, einer Creme, eines Gels oder eines Schaums vorliegt.

13. Zusammensetzung nach einem der vorhergehenden Ansprüche, dadurch gekennzeichnet, daß es sich um ein wässeriges nachschäumendes Rasiergel handelt, das ferner ein flüchtiges schäumendes Mittel 〈〈 mit verzögerter Wirkung 〉〉 umfaßt, das aus der Gruppe der Butane, Pentane, Hexane und der Gemische dieser Verbindungen ausgewählt ist.

14. Gel nach Anspruch 13, dadurch gekennzeichnet, daß das flüchtige nachschäumende Mittel ein Gemisch von Isopentan und Isobutan ist.

15. Gel nach Anspruch 13 oder 14, dadurch gekennzeichnet, daß das nachschäumende Mittel in Konzentrationen von 0,5 bis 10 Gew.-% und vorzugsweise von 2 bis 5 Gew.-% vorliegt, bezogen auf das Gesamtgewicht der Zusammensetzung.

16. Gel nach den Ansprüchen 12 bis 15, dadurch gekennzeichnet, daß es unter Druck in Form eines Aerosols vorliegt.

17. Verwendung einer Zusammensetzung nach einem der vorhergehenden Ansprüche zum Rasieren des Körperhaars oder des Kopfhaars.

18. Verwendung einer Zusammensetzung nach einem der Ansprüche 1 bis 12 als desodorierende Zusammensetzung oder zur Herstellung einer desodorierenden Zusammensetzung.
